# EUROPEAN PATENT SPECIFICATION

(11) **EP 2 051 963 B1**
(45) Date of publication and mention of the grant of the patent: **16.05.2012**
(21) Application number: 07837002.0
(22) Date of filing: 17.08.2007
(51) Int. Cl.: C07D 211/22

(54) **PROCESS FOR RESOLVING CHIRAL PIPERIDINE ALCOHOL AND PROCESS FOR SYNTHESIS OF PYRAZOLO [1,5-A]PYRIMIDINE DERIVATIVES USING SAME**
VERFAHREN ZUR LÖSUNG VON CHIRALPIPERIDINALKOHOL UND VERFAHREN ZUR SYNTHESE VON PYRAZOL[1,5]-PYRIMIDIN-DERIVATEN MIT DIESEM VERFAHREN
PROCÉDÉ DE RÉSOLUTION D'UN PIPÉRIDINE-ALCOOL CHIRAL ET PROCÉDÉ DE SYNTHÈSE DE DÉRIVÉS PYRAZOLO[1,5-A]PYRIMIDINE À L'AIDE DE CELUI-CI

(30) Priority: 18.08.2006 US 838691 P
(43) Date of publication of application: 29.04.2009
(73) Proprietor: Schering Corporation, Kenilworth, NJ 07033 (US)
(72) Inventor: CHEN, Frank, Xing, Plainsboro, NJ 08536 (US); TAMAREZ, Maria, M., Rahway, NJ 07065 (US); XIE, Ji, Edison, NJ 08817 (US)
(74) Representative: Hussain, Deeba
(86) International application number: PCT/US2007/018289
(87) International publication number: WO 2008/021509

(56) References cited:
- US-A1- 2004 209 878
- US-A1- 2004 210 060
- TOY; PRICE: "d- and l-Polyconidine" JOURNAL OF THE AMERICAN CHEMICAL SOCIETY, vol. 82, 1960, pages 2613-2616, XP002464237 cited in the application

## Description

### Field of the Invention

This application discloses a novel process to resolve mixtures of enantiomers of 2-Piperidin-2-yl-ethanol and its use in the preparation of 3-alkyl-5-piperidin-1-yl-3,3a-dihydro-pyrazolo[1,5-a]pyrimidin-7-yl)-amino derivatives, which have utility, for example, as pharmaceutically active compounds having CDK2 inhibitor activity.

### Background of the Invention

Identification of any publication in this section or any section of this application is not an admission that such publication is prior art to the present invention.

The preparation of compounds of Formula I, for example, the compounds of Formula III, has been described in Published U.S. Patent Application No. 2004-0209878 A1, filed on February 11, 2004 (the '878 publication), which is incorporated herein by reference. 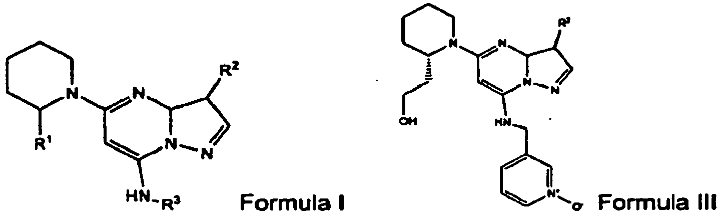 wherein R¹ is a linear, branched, or cyclic alkyloxy functional group of the structure (-R^{2a}-OH), R^{2a} is a linear, branched or cyclic alkylene group, R² is a linear, branched or cyclic alkyl group, and R³ is an alkylene-heterocycle, for example, the 3-methylene-pyridine-N-oxide substituent shown in the structure of Formula III. These compounds are believed to have pharmaceutical activity as compounds having cyclin-dependent kinase inhibitor (CDK inhibitor) properties.

As described in the '878 publication, the compounds of Formula I can be prepared through the general routes described below in Scheme I. 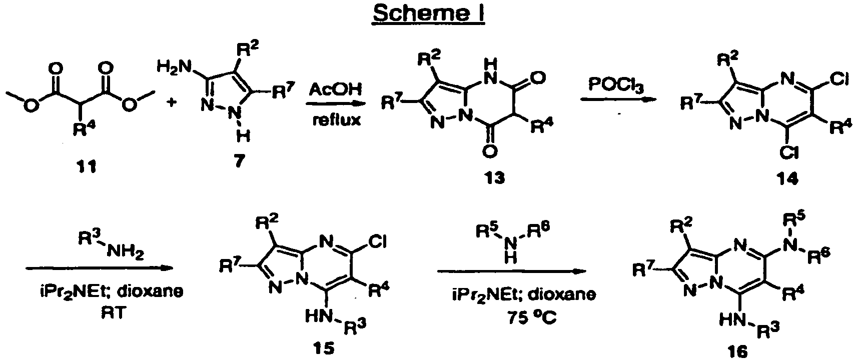 Where R¹, R². and R³ are as defined above, R⁴ and R⁷ are H, or R² and R⁵ and R⁶ are taken together to form an alkyl heterocycle, for example, pyrimidin-1-yl, optionally substituted on any carbon with a linear, branched, or cyclic alkyl, which is optionally substituted with hydroxide.

One of these inhibitor compounds of particular interest for its CDK inhibiting activity is the compound of Formula II. 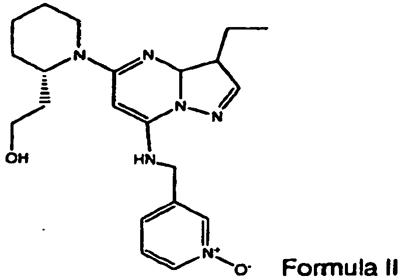 As described in detail in the '878 publication, in Example 500, on pages 334 to 343, the compound of Formula II can be synthesized in accordance with Scheme II:

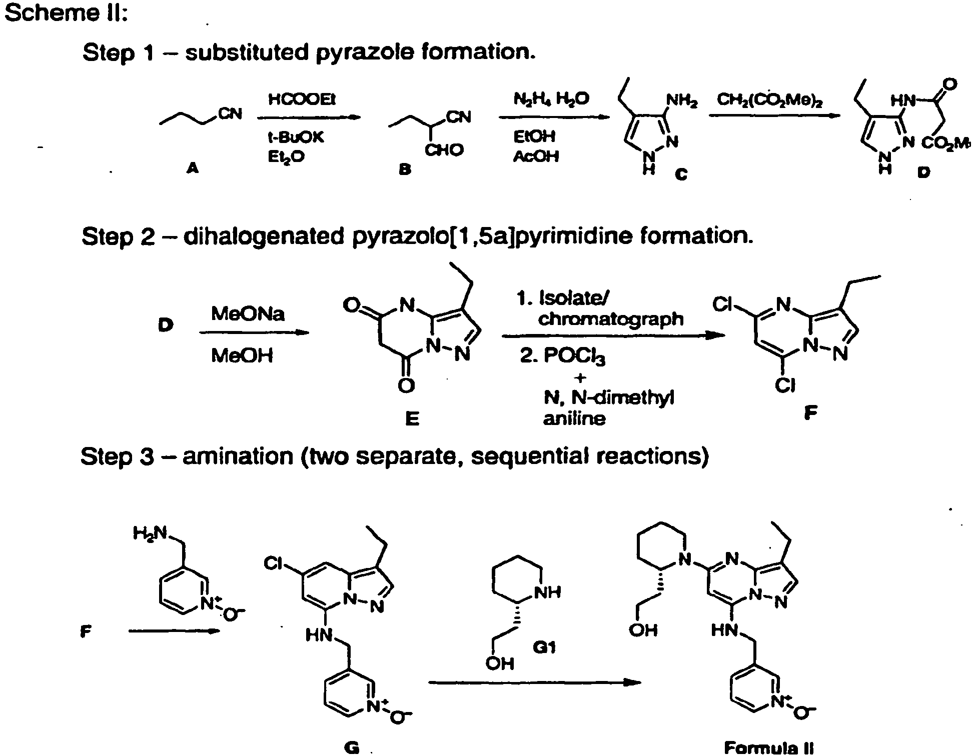

In the second amination reaction of Step 3 of the Scheme II synthesis, the chiral alcohol reagent (S)2-piperidin-2-yl ethanol (compound G1) is reacted with (5-Chloro-3-ethyl-pyrazolo[1,5-a]pyridin-7-yl)-(1-oxy-pyridin-3-ylmethyl)-amine (compound G) to yield the compound of Formula II.

The chiral alcohol (G1) is commercially available as a mixture of R and S isomers. Resolution of these isomers has been accomplished by complexing the alcohol with d-10-charnphorsulfonic acid (the compound of the structure of Formula G2a) and crystallizing the complexed alcohol from ethanol in accordance with a literature procedure published in the Journal of the American Chemical Society, 82, pp 2613 to 2616 (1960). 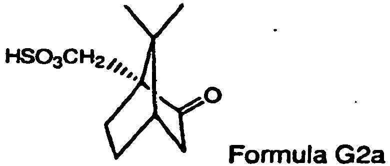 Resolution of 2-piperidin-2-yl ethanol by this method has also been reported in the Journal of Medicinal Chemistry, 45, p 2432, Bioorganic and Medicinal Chemistry Letters, 10, 1732 (2000), Chirality, 10, p 434 (1998), and the Journal of the Chemistry Society, Perkin Transactions I, 63, p 2903 (1994). This published procedure generally provides the alcohol in a yield of less than 17% based on the mixed isomer starting alcohol, and typically produces the desired isomer in yields of less than 10% based on the starting alcohol. When using this procedure, generally numerous sequential recrystallizations are required to achieve enantiomeric purity of 95 % enantiomeric excess (ee) or more, which diminishes the yield of the desired isomer. Enantiomeric purity is calculated by subtracting the weight of the minority isomer from the weight of the predominant isomer present in a sample, and dividing the difference by the sum of the minority and predominant isomers present in the sample. Accordingly, a sample having 97.5 wt.% of the S isomer and 2.5 wt.% of the R isomer has an enantiomeric purity of 95% ee. In the synthesis of CDK inhibitor compounds described above, it is desirable to utilize the desired isomer of piperidine-ethanol starting materials having more than about 97% ee purity.

### Objectives

In view of the foregoing, what is needed is a resolution process for preparing the critical intermediate 2-piperidin-2-yl ethanol useful in the synthesis of CDK inhibitor compounds that provides the alcohol in high enantiomeric purity, high chemical purity, and in yields of more than about 17 %. These and other objectives and/or advantages are provided by the present invention.

### Summary of the Invention

In one aspect the present invention is a process for providing 2-piperidin-2-yl ethanol in an enantiomeric excess (ee) of at least about 95% utilizing a single crystallization step, the process comprising:
(a) combining a solution comprising a polar, aprotic organic solvent and a mixture of isomers of 2-piperidin-2-yl-ethanol with an aliphatic alcohol solution containing an amino acid resolving agent selected from N-acetyl-L-leucine and N-acetyl-L-methionine;
(b) mixing the combined solutions with additional amounts of the polar, aprotic organic solvent and warming the mixture to a temperature below the boiling point of either solvent in the mixture sufficient to promote dissolution of any solids present in the mixture;
(c) cooling the mixture to a temperature which permits salt precipitation therefrom;
(d) optionally isolating the precipitated salt formed in step "c"; and
(e) optionally purifying the isolated salt from Step "d" by recrystallization or slurrying with a purifying solvent.

In some embodiments it is preferred for the polar, aprotic organic solvent in Step "a" to be selected from tetrahydrofuran (THF), acetonitrile (ACN), acetone, and ethylacetate, more preferably the polar, aprotic organic solvent is THF. In some embodiments it is preferred to select the aliphatic alcohol in Step "a" from alcohols having 5 carbon atoms or less, more preferably, the aliphatic alcohol is methanol. In some embodiments it is preferred to use a volumetric ratio of polar aprotic organic solvent (PAO solvent): aliphatic alcohol of from about 20:1 PAO solvent:aliphatic alcohol to about 2:1 PAO solvent:aliphatic alcohol, more preferably from about 19:1 PAO solvent:aliphatic alcohol to about 5:1 PAO solvent:aliphatic alcohol. In some embodiments it is preferred to use a ratio of about 19:1 PAO solvent:aliphatic alcohol. In some embodiments it is preferred to usa a ratio of about 5:1 PAO solvent: aliphatic alcohol.

In some embodiments of the present invention, purification step "e" is a recrystallization carried out in a mixed solvent, preferably a mixture of acetonitrile (ACN) and methanol (MeOH) selected with a ratio of the component suitable for dissolving the salt at a temperature above ambient and precipitating the salt when the temperature is reduced, preferably to a point below ambient. In some embodiments the purification step "e" is conducted by slurrying the precipitate provided in step "d" with a solvent in which the unwanted constituents are soluble but the desired salt is not soluble to any great extent, thereby leaving the desired salt undissolved thereby separating the desired salt and the impurities coprecipitated in step "d". When purification Step "e" constitutes slurrying, preferably a solvent mixture is used having a volumetric ratio of from about 10:1, ACN:MeOH to a volumetric ration of about 20:1 ACN:MeOH. In some embodiments purification step "e" is repeated until the desired ee value is obtained.

In some embodiments of the invention which include a precipitation step "d" the process further includes isolation of the precipitated salt from step "d", redissolving, or partially redissolving the salt in a solvent and liberating the alcohol free base prior to using the alcohol in a reaction by treatment of the solution or slurry with a base. In some embodiments which include a purifying step "e" the process further includes isolating the purified salt from step "e", redissolving or partially dissolving the salt and liberating the alcohol free base prior to using the alcohol in a reaction. In some embodiments of the invention including a precipitation step "d" or a precipitation step "d" followed by a purification step "e", the process further includes utilizing the salt produced in step "d" or in step "e" directly in a reaction and liberating the alcohol free base therefrom *in situ* in the reaction mixture.

In some embodiments of the present invention it is preferred to use N-acetyl-L-leucine as the resolving agent. In some embodiments of the invention, it is preferred to use a methanol solution having a resolving agent concentration which is at least 1 M in resolving agent, more preferably from about 3 M to about 10 M in resolving agent, and more preferably about 5 M in resolving agent. In some embodiments of the invention it is preferred to provide a THF solution in step "a" of the resolving process having a concentration of 2-piperidin-2-yl-ethanol which is at least about 0.05 M, more preferably from about 1 M to about 5 M, and more preferably has about a 2 M concentration in 2-piperidin-2-yl-ethanol.

In some embodiments it is preferred to maintain the methanol solution and the combined methanol and THF solutions in step "a" at a temperature of from about 35 °C to about 40 °C. In some embodiments it is preferred in step "b" of the resolution process to warm the combined and diluted solutions to a temperature of from about 50 °C to about 55 °C. In some embodiments of the invention it is preferred to cool the solution in step "c" to a temperature of at least 15 °C. more preferably to cool the solution to a temperature of from about 10 °C to about 15 °C.

In some embodiments utilizing purification step "e" it is preferred to slurry the precipitate from step "d" by heating a mixture of the precipitated salt from step "d" with a mixed solvent comprising about 1 vol. eq. of methanol and about 20 vol. eq. of acetonitrile to a temperature sufficient to partially dissolve the salt, preferably a temperature of about 55 °C, and cooling it to a temperature of about 15 °C to precipitate purified salt.

In one aspect the present invention is part of a process for the provision of pyrazolo[1,5-a]pyrimidin-7-yl-amino compounds of Formula III having CDK inhibitor properties: 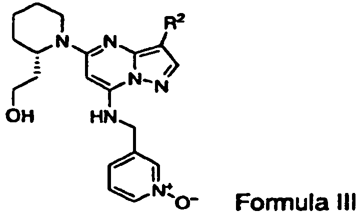 wherein R² is a linear, branched or cyclic alkyl group, the process comprising:
(a) combining a solution comprising a polar, aprotic organic solvent and a mixture of isomers of 2-piperidin-2-yl-ethanol with an aliphatic alcohol solution containing an amino acid resolving agent selected from N-acetyl-L-leucine and N-acetyl-L-methionine;
(b) mixing the combined solutions with additional amounts of the polar, aprotic organic solvent and warming the mixture to a temperature below the boiling point of either solvent in the mixture sufficient to promote dissolution of any solids present in the mixture;
(c) cooling the mixture to a temperature which permits the salt of compound G1a to precipitate therefrom, 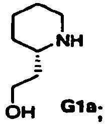
(d) purifying the salt from step "c" by a technique selected from slurrying the salt and recrystallizing the salt;
(e) liberating the free base alcohol G1a from the salt prepared in purifying Step "d"; and
(f) reacting the liberated free base alcohol from Step "e" with the compound of Formula G, 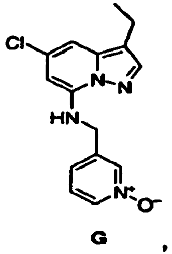 to yield a compound of Formula III.

In some embodiments it is preferred for the polar, aprotic organic solvent in Step "a" to be selected from tetrahydrofuran (THF), acetonitrile (ACN), and acetone, more preferably the polar, aprotic organic solvent is THF. In some embodiments it is preferred to select the aliphatic alcohol in Step "a" from aliphatic alcohols having 5 carbon atoms or less, more preferably, the aliphatic alcohol is methanol.

In some embodiments of the present invention, purification step "d" is carried out in a mixed solvent, preferably a mixture comprising a polar aprotic solvent selected from THF, ACN, and acetone and an aliphatic alcohol, more preferably a mixture comprising acetonitrile (ACN) and methanol (MeOH). In some embodiments it is preferred to use a mixture having a 10:1 volumetric ratio of ACN:MeOH. In some embodiments the purification step "d" is repeated until the desired ee value is obtained.

In some embodiments liberating step "e" is carried out by treating the salt in a separate solution with a base, preferably a base selected from potassium hydroxide and sodium hydroxide, thereby precipitating the free base which is isolated and added to a reaction medium in reaction step "f". In some embodiments, the liberating step "e" is carried out *in situ* within a reaction medium and contemporaneously reacted with a compound of Formula G.

These and other aspects of the invention will become apparent from the following detailed description.

### Detailed Description of the Invention

As mentioned above, and described in the '878 publication, compounds of Formula I (as defined herein) are believed to have promising activity as useful pharmaceutical compounds having CDK inhibitor properties. A synthesis of these compounds, for example, the compound of Formula II, is described in detail in published U.S. Patent Application No. 2004-0209878 A1, filed on February 11, 2004 (the '878 publication). A process for providing the compound of Formula II is described in the '878 publication in Examples 507 to 508, 509, 1000 and 1001. These examples, as well as the entirety of the '878 publication are incorporated herein by reference. It will be appreciated that the present invention method for resolving chiral amino alcohol can be used in conjunction with any synthetic process requiring the use of a selected isomer of the alcohol. As mentioned above, and in the cited documents, (S)2-piperidin-2-yl-ethanol (the compound of Formula G1) is a critical intermediate in the in synthetic pathway for the preparation of compounds of Formula I, Formula II, and Formula III described in each of the aforementioned documents. The inventors have surprisingly discovered a resolving method for providing a high yield of the desired isomer in enantiomeric purity exceeding about 95% ee in a resolution process utilizing a single crystallization step. Moreover, if additional recrystallization steps are added the inventive process yields higher % ee material with recrystallized product that exceed 95% based on the starting amount of resolved alcohol recrystallized.

As used herein to describe the present invention the following terms have the following meanings.

Alkyl- is a linear, branched, or cyclic hydrocarbon substituent having a single point of attachment between the group and the substrate upon which it is a substituent. When the term Alkylene, for example methylene (-CH₂-), ethylene (-CH₂CH₂-), etc. is used, it is a linear branched or cyclic hydrocarbon substituent having two points of attachment, for example, as a bridge between functional groups.

The method of the present invention comprises providing separately a solution comprising the alcohol to be resolved (an amino alcohol) dissolved in a polar, aprotic organic solvent (PAO solvent) and a solution comprising the resolving agent dissolved in an aliphatic alcohol which are combined, heated and mixed with an additional amount of PAO or an amount of a solvent selected from tetrahydrofuran and acetonitrile or mixtures thereof, and then cooled to precipitate a complex of the resolving agent and the desired isomer of the amino alcohol which is being resolved. Subsequently, the precipitated complex, optionally purified by recrystallization or slurrying, is treated to liberate the free base form of the desired isomer of the resolved amino alcohol which is employed in further reactions, most usefully in the preparation of CDK inhibitor compounds. Optionally, the resolved amino alcohol complex prepared by the inventive method is used, either in "as-precipitated" form or in purified form, by introducing it directly into the reaction mixture of a subsequent reaction and liberating its free base *in situ* in the reaction medium.

The complex formation/precipitation step is followed by isolating the resolving agent/alcohol complex and optionally purifying the isolated complex by recrystallization or using a slurry technique. When employed, recrystallization to purify the isolated complex is carried out using a mixed solvent system of different polarity than the solvent system in which the alcohol/resolving agent complex was formed. Surprisingly, by using this methodology, higher enantiomeric purity (ee) resolved product is produced with the same number of recrystallizations when compared to prior art methods. This has the benefit of providing a high enantiomeric purity product in higher yield base on the starting alcohol to be resolved than is available with other methods. Each aspect of the methodology will be discussed next.

### Resolving Agent

The present resolution method utilizes as a resolving agent either N-acetyl-L-leucine (the compound of the structure of Formula G2b) 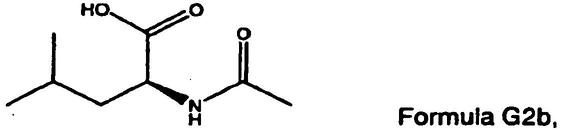 or N-acetyl-methionine (the compound of the structure of Formula G3b, 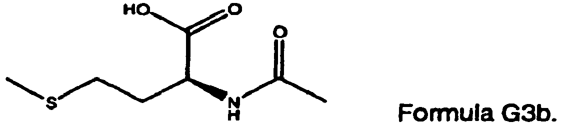 to form a complex with the amino alcohol to be resolved. Without being bound by theory, it is believed that the complex formed using these two resolving agents results in a greater change in polarity when it complexes with the desired amino alcohol isomer than it does when it complexes with the undesired amino alcohol isomer and/or has a higher selection for complexing with the desired amino alcohol isomer than with the undesired amino alcohol isomer, thus permitting the alcohol/resolving agent complex to be precipitated from a mixed solvent having lower polarity than the neat aliphatic alcohol comprising the mixed solvent while permitting a higher proportion of the complex of the desired isomer to be precipitated therefrom relative to the undesired isomer.

### The Amino Alcohol to be Resolved

While it is believed that the method of the present invention will be most beneficial when applied to isolating in high yield and high enantiomeric purity (S)2-piperidin-2-yl-ethanol from a mixture of the "R" and "S" 2-piperidin-2-yl-ethanol isomers, other related piperidine alcohols may also be resolved using the method, for example those having the structure of Figure G4c, wherein the 2-ethanol substituent can be attached to the piperidine ring at any of the 2, 3, or 4-carbon atom position, and those having the structure of Figure G5c, 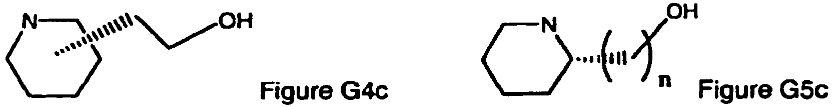 where "n" is an integer of 1 to 4. The method is also believed to be useful for resolving 2-piperidin-2-yl-ethanol compounds of either the structure of Figure G4c or of Figure G5c having substituents on other carbon atoms of the piperidine ring in addition to the (S)2-ethanol substituent shown, for example, liner, branched or cyclic alkyl groups having less than 4 carbon atoms.

### Complex Formation/Precipitation Step

The mixed solvent in which the piperidine alcohol/resolving agent complex is prepared in accordance with the present invention methodology comprises an aliphatic alcohol and a polar aprotic organic solvent (also termed herein for convenience "PAO" solvent) having less polarity than the aliphatic alcohol selected, in a ratio that provides a mixed solvent of less proton doner ability and lower polarity than the selected aliphatic alcohol alone. Accordingly, on a volumetric basis, suitable mixtures of polar aprotic organic solvent (PAO solvent) and aliphatic alcohol comprise from about 2:1 PAO solvent:aliphatic alcohol to about 10:1 PAO solvent:aliphatic alcohol, more preferably mixtures comprising a volumetric ratio of about 5:1 PAO solvent:aliphatic alcohol. In general, after mixture of the piperdine alcohol and resolving agent, the PAO solvent will act as an antisolvent and the aliphatic alcohol will act as a solvent of the salt product. Accordingly, the ratio of the PAO solvent and aliphatic alcohol are selected to strike a balance of the amount of salt product retained in solution and the amount of unwanted isomer coprecipitated with the salt product of the desired isomer. Accordingly, ratio's of aliphatic alcohol and PAO solvent lying outside of these ranges may also be used.

Examples of suitable polar, aprotic organic solvents which may be employed in the present invention resolution method include acetonitrile (ACN, Snyder solvent polarity index 6.2), acetone (Snyder solvent polarity index 5.4), ethyl acetate (Snyder solvent polarity index 4.3), tetrahydrofuran (THF, Snyder solvent polarity index 4.2) and mixtures of 2 or more of these. For resolution of (S)-2-piperidin-2-yl-ethanol, it is preferred to use THF as the polar, aprotic organic solvent.

Examples of aliphatic alcohols which may be employed in the present invention resolution method include R-OH in which "R" is a linear, branched, or cyclic alkyl group of 5 carbon atoms or less, preferably methanol (Snyder solvent polarity index 6.6), 1-propanol, 2-propanol (Snyder solvent polarity index 4.3), and ethanol (Snyder solvent polarity index 5.2). In general it is preferred to employ aliphatic alcohols having lower pKa values, for example, methanol (pKa about 15.5) and ethanol (pKa about 16) than less acidic alcohols, for example i-propanol (pKa about 16.5). In general it is preferred to employ alcohols having higher polarity on the Snyder polarity scale, for example methanol and ethanol, more preferably methanol is employed.

The amount of polar, aprotic organic solvent employed in the resolution process of the invention will be an amount sufficient to dissolve the amount of amino alcohol to be resolved. In some embodiments of the invention it is preferred to provide a PAO solvent solution in the complex formation/precipation step of the resolving process having a concentration of the alcohol to be resolved which is at least about 0.05 M, more preferably from about 1 M to about 5 M, and more preferably has about a 2 M concentration of the amino alcohol to be resolved. When the alcohol to be resolved is 2-piperidin-2-yl-ethanol it is preferred to supply a solution of THF containing 2M concentration of 2-piperidin-2-yl-ethanol to the complex formation/precipitation step. Within these guidelines, the PAO solvent solution of amino alcohol to be resolved may be heated to effect dissolution within these guidelines.

Guided by the above-mentioned desired volumetric ratios of PAO:aliphatic alcohol, the amount of aliphatic alcohol used to provide a solution of resolving agent to the complex formation/precipitation step of the process of the invention will be at least sufficient to dissolve the resolving agent employed. It is preferred to use sufficient aliphatic alcohol to provide a solution which has a resolving agent concentration that is at least 1 M in resolving agent, more preferably from about 3 M to about 10 M in resolving agent, and more preferably about 5 M in resolving agent. Within these general guidelines, the aliphatic alcohol solution may be heated to effect dissolution of the resolving agent.

When the method of the present invention is used to resolve (S) 2-piperidin-2-yl-ethanol, it is preferably to heat the resolving agent and amino alcohol solutions to a temperature of at least 35°C prior to combining the solutions and to maintain the temperature upon combining the solutions at a temperature of from about 35 °C to about 40 °C.

Upon combination of the resolving agent aliphatic alcohol solution and the PAO solvent solution of amino alcohol to be resolved, turbidity in the mixed solutions may occur. After combination of the resolving agent and amino alcohol to be resolved, additional solvent is added to solubilize the solids produced when combining the solutions, and the mixture is heated. In general, the added solvent is selected from the PAO solvent used to dissolve the amino alcohol, however, other PAO solvents can be employed also. Preferably the PAO solvent is selected from thTHF and ACN, more preferably the less polar THF is selected.

After adding additional solvent, the mixture is heated, preferably to a temperature of at least about 50 °C, but not more than the boiling point of the solvent selected. When THF is selected as the additional solvent added in this step, preferably the temperature maintained from about 50 °C to about 55 °C.

After all of the solids have dissolved the solution is held at a temperature of from about 50 °C to about 55 °C for 30 minutes. The mixture is then cooled to subambient temperature to precipitate the complex, generally gradually, to control the size of the crystals precipitated from the mixture and minimize included solvent in the precipitate. When the method is utilized to prepare (S) 2-piperidin-2-yl-ethanol the cooling step is carried out over a period of about 2 hours, reducing the temperature of the mixture from a temperature of about 50 °C to about 55°C to a temperature of about 15°C.

In another aspect, the present invention process forms a part of an improved synthesis for the preparation of pyrazolo-[1,5-a]-pyrimidine compounds carried out in accordance with Scheme III. 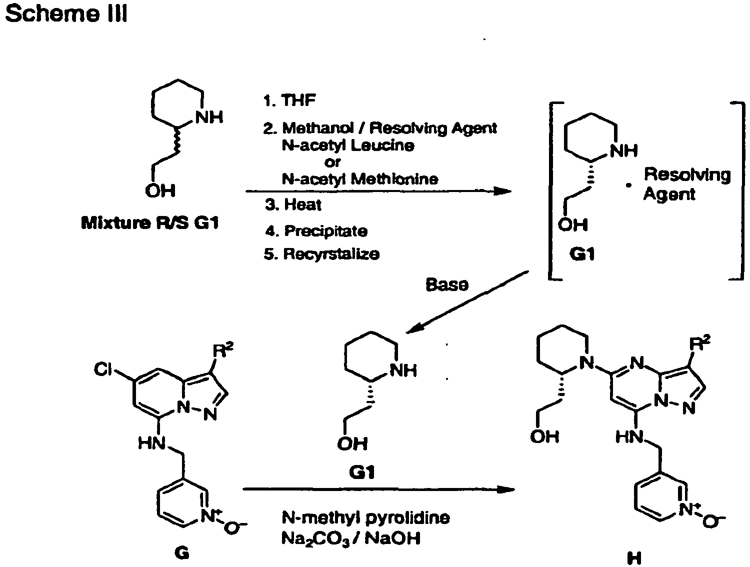 wherein R² is a linear, branched or cyclic alkyl group, and X is selected from a halogen and a sulfonyl leaving group. Preferably R² has 4 carbon atoms or fewer, more preferably R² is an ethyl- group (-CH₂CH₃). Preferably X is chloride

It will be appreciated that the present invention resolution process can be used to supply the resolved 2-piperidin-2-yl-ethanol intermediate and reacted with compounds of Formula G prepared by any known method of supplying compounds of Formula G. Particularly preferred are reaction schemes described in the above-referenced '878 publication. In some embodiments of the invention, preferably the resolution scheme of the present invention is combined with reaction schemes producing the compound of Formula III.

It will be appreciated that the above-described Scheme III can be used to prepare compounds of Formula I, Formula II, and Formula III, depending upon the structure of the (5-Chloro-3-alkyl-pyrazolo[1,5-a]pyridin-7-yl)-(1-oxy-pyridin-3-ylmethyl)-amine employed. Particularly preferred are reactions in which the R² group of the compound of Formula G is an ethyl- group and X is chloride, thereby providing the compound of Formula G1a, which yields a product of the structure of Formula II described above.

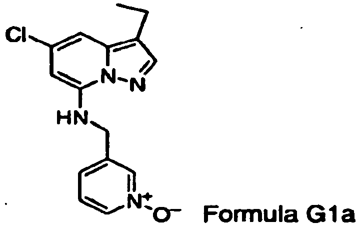

A particularly preferred scheme for preparing a compound of Formula II is shown in Scheme IV, below. It is believed that the present invention method of resolving (S) 2-piperidin-2-yl-ethanol will be especially useful when used in conjunction with Scheme IV because in step 3, the second amination reaction, which utilizes the (S) isomer of 2-piperidin-2-yl-ethanol, is performed in the presence of sodium or potassium hydroxide. Accordingly, in this step the compound of Formula G1 can be provided to the reaction mixture in the form of its recrystallized salt, or in the form of the precipitated product from the previous step, which will be converted *in situ* to the free base, without requiring a separate step for liberating the free base prior to employing it in a reaction.

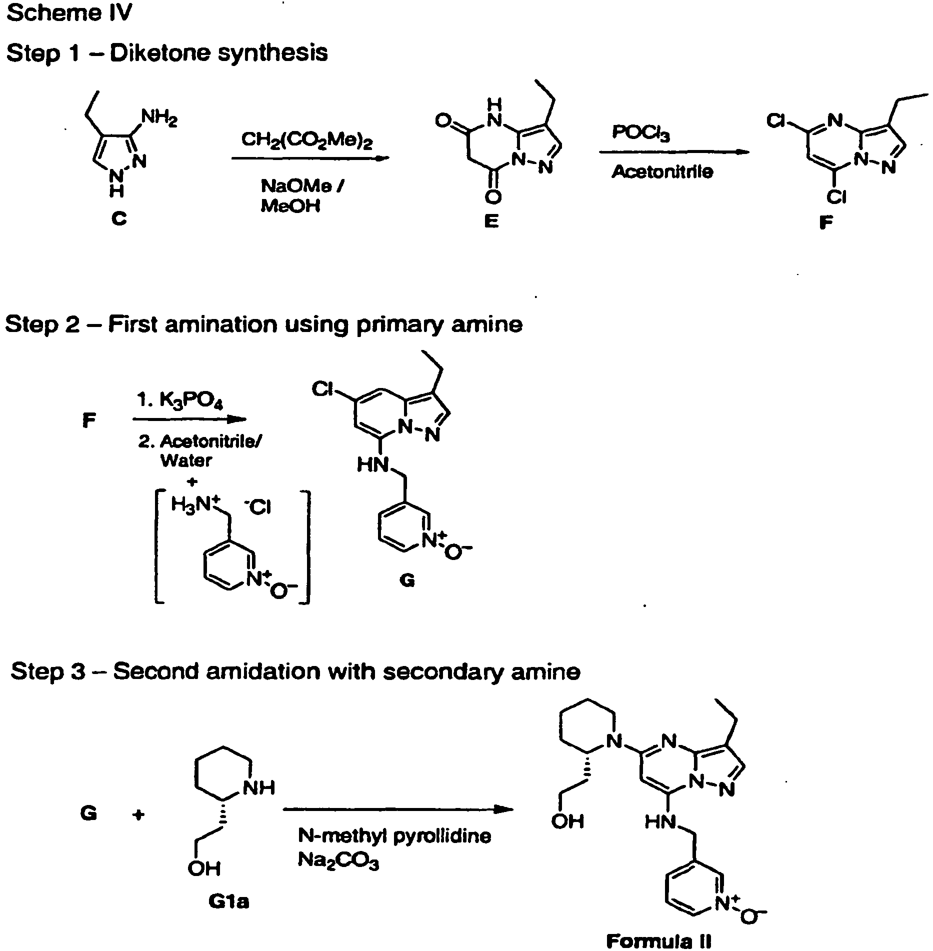

### EXAMPLES

The following solvents and reagents may be referred to by their abbreviations in parenthesis:
tetrahydrofuran : THF
methanol : MeOH
HPLC : high pressure liquid chromatography
mole: mol.
ee : enantiomeric excess - (weight of desired enantiomer recovered)/ (weight of desired enantiomer present + weight of undesired enantiomer present)

All reagents were obtained from Aldrich or Acros and used as received unless otherwise noted.

### Example 1: Preparation of Preparation of (S)-2-Piperidin-2-yl-Ethanol

Into a flask was placed N-acetyl-L-Leucine (8.65 g) dissolved in 10 ml of methanol. The solution was heated, maintaining the temperature at from 35 °C to 40 °C with stirring. A tetrahydrofuran (THF) solution prepared from 50 ml of THF and 12.9 g (0.1 mol.) of 2-piperidine-2-yl-ethanol (mixture of R and S isomers) was added to the methanol solution maintaining the temperature-of the combined solutions at 35 °C to 40 °C. An additional 30 ml of THF was added and the resulting mixture was heated and maintained at a temperature of from 50 °C to 55 °C for 30 minutes. The reaction mixture was cooled over two hours to 15 °C and held at that temperature for one hour, during which time salt precipitated. The precipitate was recovered by vacuum filtration and dried under vacuum at ambient temperature (about 25 °C). The recovered precipitate was tested for ee purity by HPLC through derivatization of the product with benzoyl chloride and found to be 94.5% ee S-isomer. A yield of 37.7% based on the weight of the unresolved alcohol starting material was calculated (11.4g isolated S-isomer), accordingly a loss of about 25% S-isomer.

### Purification of Precipitate

An aliquot of the precipitate salt thus formed (7.0 g) was suspended with stirring in a solvent comprising 50 ml of acetonitrile and 2.5 ml of methanol by heating the mixture to 55 °C and held for 30 minutes. The resultant suspension was cooled over a period of 2 hours to a temperature of 15 °C. The resulting crystals were obtained by filtration and tested by HPLC for purity and a yield was calculated based on recovered weight. The precipitation yielded 6.7 g of precipitate (calculated yielded 95.7% based on starting precipitated complex) and had a isomeric purity of 97.7% ee based on HPLC analysis.

### Conversion to Free Base

A salt of the piperidine ethanol is converted to the free base by dissolving 13 mmol. of the purified salt in 24 ml of 3N NaOH and stirring the resulting solution vigorously for about 1.5 hours. At the end of 1.5 hours of stirring, 7.5ml of water is added to the solution. This mixture is then extracted with methylene chloride three times. The methylene chloride extracts are concentrated to yield approximately 13 mmol. of free piperidine ethanol. The chiral purity of the product is found to be the same as that of the original salt.

### Comparative Example

The stereoisomer of the 2-Piperidin-2-yl-ethanol (Compound G1a) was prepared as described in the '878 publication in accordance with preparative Example 500, therein.

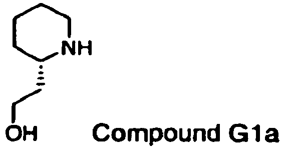

Thus, a mixture of R and S enantiomers of piperidine-2-ethanol obtained from Acros and used as received (127 g, 980 mmol) was dissolved in 95% EtOH (260 mL). To the aliphatic alcohol solution was added to (S)-(+)-camphorsultonic acid obtained from Acros (228.7 g, 1.0 eg.) in 95% EtOH (150 mL) and the resulting solution was warmed to reflux. To the warm solution was added Et₂O (600 mL) and the solution cooled to room temperature and let stand 3 days. The resulting crystals were filtered and dried *in vacuo* (25 g): and analyzed by mp 173 °C (lit. 168 °C). The salt was then dissolved in NaOH (3M, 100 mL) and stirred 2 hours and the resulting solution was extracted with CH₂Cl₂ (5 x 100 mL). The combined organics were dried over Na₂SO₄, filtered and concentrated under reduced pressure to give (*S*)-piperidine-2-ethanol (7.8 g) a portion of which was recrystallized from Et₂O: mp= 69-70 °C (lit. 68-69 °C); [α]_{D} = 14.09° (CHCl₃, c=0.₂). Overall yield of S-isomer isolated, based on initial weight of starting alcohol was 6. 1 %.

## Claims

1. A process for resolving the S-isomer of 2-piperidin-2-yl ethanol from a mixture of isomers, the process comprising:
(a) combining a solution comprising a polar, aprotic organic solvent and a mixture of isomers of 2-piperidin-2-yl-ethanol with an aliphatic alcohol solution containing an amino acid resolving agent selected from N-acetyl-L-leucine and N-acetyl-L-methionine;
(b) mixing the combined solutions with additional amounts of the polar, aprotic organic solvent and warming the mixture to a temperature below the boiling point of either solvent in the mixture sufficient to promote dissolution of any solids present in the mixture;
(c) cooling the mixture to a temperature which permits salt precipitation therefrom;
(d) optionally isolating the precipitated salt formed in step "c"; and
(e) optionally purifying the isolated salt from Step "d" by recrystallization or slurrying with a purifying solvent.

2. The process of claim 1 wherein the polar aprotic solvent used in said "combining" Step "a" is a solvent selected from tetrahydrofuran (THF), acetonitrile (ACN), acetone, and ethylacetate, and mixtures of two or more thereof.

3. The process of claim 2 wherein said polar aprotic solvent is THF.

4. The method of any of claims 1 to 3 wherein, said recrystallization step "e", when carried out, is carried out in a mixed solvent.

5. The method of claim 4 wherein said mixed solvent is a mixture of a PAO solvent selected from acetonitrile and THF and an aliphatic alcohol having 5 or fewer carbon atoms.

6. The method of claim 5 wherein, said mixture comprises 19 volumes of acetonitrile and 1 volume of methanol.

7. The method of claim 1 wherein said recrystallization step "e", when preformed, is repeated until the desired enantiomeric purity is achieved.

8. The method of any of claim 1 further comprising the steps of:
(a) providing a mixture comprising redissolving or suspending the precipitated salt in a solvent; and
(b) treating the mixture from step "a" with a base to yield (S)2-piperidine-2-yl-ethanof free base.

9. The resolution process of any of claims 1 to 8 wherein the precipitated salt is introduced into a reaction mixture containing a base, thereby providing (S)2-piperidine-2-yl-ethanol free base *in situ* in the reaction mixture.

10. The resolution process of any of claims 1 to 9, wherein the resolving agent selected for use in step "a" is N-acetyl-L-leucine.

11. The resolution process of any preceding claims wherein the resolving agent is present in the step "a" solution in an amount sufficient to provide a 5 M solution of resolving agent.

12. The resolution process of any preceding claim wherein the mixture of solvents used in Step "a", the combining step, comprises a volumetric ratio of polar aprotic organic solvent (PAO solvent): aliphatic alcohol of from about 2:1 PAO solvent:aliphatic alcohol to about 10:1 PAO solvent: aliphatic alcohol.

13. The resolution process of claim 12 wherein the mixture of solvents used in comprises a volumetric ratio of about 5:1 PAO solvent:aliphatic alcohol

14. The resolution process of any preceding claim where the PAO solvent is THF and comprises a solution that contains an amount of 2-piperidin-2-yl-ethanol sufficient to provide a solution which is at least 2 M in2-piperidin-2-yl-ethanol.

15. The resolution process of any preceding claim wherein the combined methanol and THF solutions in step "a" are maintained at a temperature of from about 20 °C to about 60 °C.

16. The resolution process of any preceding claim wherein the combined methanol and THF solutions in step "a" are maintained at a temperature of from about 35 °C to about 40 °C.

17. The resolution process of any preceding claim wherein the combined and diluted solution of step "b" is heated to a temperature of from about 30 °C to a temperature below the refluxing temperature of the solvent mixture.

18. The resolution process of any preceding claim wherein the combined and diluted solution of step "b" is heated to a temperature of from about 50 °C to about 55 °C.

19. The resolution process of any preceding claim wherein step "e" purification step is preformed by crystallizing the precipitate from step "d" by heating a mixture of 1 vol. eq. of methanol and 20 vol. eq. of acetonitrile in which the salt is dissolved to a temperature of about 55 °C and cooling it to a temperature of about 15 °C.

20. The resolution process of any preceding claim wherein step "e" purification step is performed by slurrying the precipitate from step "d" in a mixture of 1 vol. eq. of methanol and 20 vol. eq. of acetonitrile and heating the slurry to a temperature of about 55 °C, holding it at temperature for a time to dissolve the impurities, and cooling it to a temperature of about 15 °C.

21. A process for making pyrazolo[1,5-a]pyrimidin-7-yl-amino compounds of Formula III 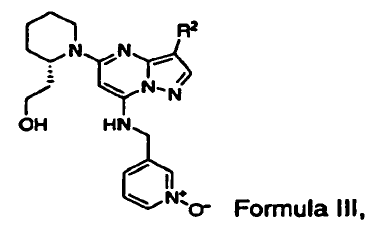 wherein R² is a linear, branched or cyclic alkyl group, the process comprising:
(a) combining a solution comprising a polar, aprotic organic solvent and a mixture of isomers of 2-piperidin-2-yl-ethanol with an aliphatic alcohol solution containing an amino acid resolving agent selected from N-acetyl-L-leucine and N-acetyl-L-methionine;
(b) mixing the combined solutions with additional amounts of the polar, aprotic organic solvent and warming the mixture to a temperature below the boiling point of either solvent in the mixture sufficient to promote dissolution of any solids present in the mixture;
(c) cooling the mixture to a temperature which permits the salt of compound G1a to precipitate therefrom, 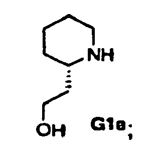
(d) isolating the precipitated salt formed in step "c";
(e) purifying the salt from step "c" by slurrying the salt in a 10:1 by volume mixture of acetonitrile (ACN) and methanol;
(f) liberating the free base alcohol G1a from the salt prepared in purifying Step "e"; and
(g) reacting the free base alcohol from Step "e" with the compound of Formula G1,

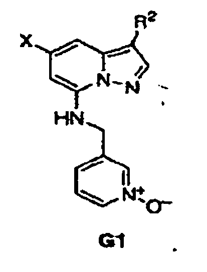

22. The process of claim 21 wherein "R²" is an ethyl- substituent and X is Cl⁻.

23. The process of any of claims 21 to 22 wherein the polar, aprotic organic solvent in Step "a" is selected from tetrahydrofuran (THF), acetonitrile (ACN), methyl acetate and acetone.

24. The process of any of claims 21 to 23 wherein the aliphatic alcohol in Step "a" is selected from aliphatic alcohols having 5 carbon atoms or less.

25. The process of any of claims 21 to 24, wherein said liberating step "f" is carried out by combining the isolated resolved salt with a reaction mixture in the presence of a base and liberating the free base alcohol *in situ.*

26. The process of any of claims 21 to 24, wherein said liberating step "f" is carried out by treating the isolated resolved salt with a base, crystallizing the free base alcohol out of solution, and isolating the free base crystals by filtration.

27. A process for resolving the S-isomer of 2-piperidin-2-yl ethanol from a mixture of isomers, the process comprising:
(a) combining a tetrahydofuran (THF) solution containing a mixture of isomers of 2-piperidin-2-yl ethanol with a methanol solution containing N-acetyl-L-leucine;
(b) mixing the combined solutions with additional THF and warming the mixture to a temperature below the boiling point of either solvent in the mixture;
(c) cooling the mixture to a temperature which permits salt precipitation therefrom;
(d) optionally isolating the precipitated salt formed in step "c"; and
(e) optionally recrystallizing the salt.

28. The method of claim 1 wherein the aprotic organic solvent is THF.

29. The method of claims 1 and 28 wherein the aliphatic alcohol used in Step "a" is an alcohol having 5 carbon atoms or less.

30. The method of claim 29 wherein the aliphatic alcohol is methanol.

31. The method of claim 6 wherein said recrystallization step "e", when preformed, is repeated until the desired enantiomeric purity is achieved.

32. The method of any of claim 31 further comprising the steps of:
(a) providing a mixture comprising redissolving or suspending the precipitated salt in a solvent; and
(b) treating the mixture from step "a" with a base to yield (S)2-piperidine-2-yl-ethanol free base

## Patentansprüche

1. Ein Verfahren zum Abtrennen des S-Isomers von 2-Piperidin-2-ylethanol von einer Isomerenmischung, wobei das Verfahren umfasst:
(a) Vereinen einer Lösung, die ein polares aprotisches organisches Lösungsmittel und eine Mischung aus Isomeren von 2-Piperidin-2-ylethanol enthält, mit einer Lösung aliphatischen Alkohols, die ein Aminosäure-Trennmittel, ausgewählt aus N-Acetyl-L-leucin und N-Acetyl-L-methionin, enthält,
(b) Vermischen der vereinten Lösungen mit zusätzlichen Mengen des polaren aprotischen organischen Lösungsmittels und Erwärmen der Mischung auf eine Temperatur, die unterhalb des Siedepunktes eines jeden Lösungsmittels in der Mischung liegt und die ausreicht, um die Auflösung jeglicher Feststoffe, die in der Mischung vorliegen, zu fördern,
(c) Abkühlen der Mischung auf eine Temperatur, die das Ausfällen von Salz aus der Mischung ermöglicht,
(d) gegebenenfalls Isolieren des in Schritt "c" gebildeten ausgefallenen Salzes und
(e) gegebenenfalls Reinigen des isolierten Salzes aus Schritt "d" durch Umkristallisieren oder Aufschlämmen mit einem Reinigungslösungsmittel.

2. Das Verfahren nach Anspruch 1, wobei das in Schritt "a", dem "Vereinschritt", verwendete polare aprotische Lösungsmittel ein Lösungsmittel ist, das aus Tetrahydrofuran (THF), Acetonitril (ACN), Aceton und Ethylacetat und Mischungen aus zwei oder mehreren davon ausgewählt ist.

3. Das Verfahren nach Anspruch 2, wobei das polare aprotische Lösungsmittel THF ist.

4. Das Verfahren nach einem der Ansprüche 1 bis 3, wobei der Umkristallisationsschritt "e", wenn er durchgeführt wird, in einem gemischten Lösungsmittel durchgeführt wird.

5. Das Verfahren nach Anspruch 4, wobei das gemischte Lösungsmittel eine Mischung aus einem PAO-Lösungsmittel, ausgewählt aus Acetonitril und THF, und einem aliphatischen Alkohol mit 5 oder weniger Kohlenstoffatomen ist.

6. Das Verfahren nach Anspruch 5, wobei die Mischung 19 Volumen Acetonitril und 1 Volumen Methanol umfasst.

7. Das Verfahren nach Anspruch 1, wobei der Umkristallisationsschritt "e", wenn er durchgeführt wird, wiederholt wird, bis die erwünschte Enantiomerenreinheit erzielt ist.

8. Das Verfahren nach Anspruch 1, ferner umfassend die Schritte:
(a) Bereitstellen einer Mischung, umfassend das Wiederauflösen oder Suspendieren des ausgefallenen Salzes in einem Lösungsmittel, und
(b) Behandeln der Mischung aus Schritt "a" mit einer Base, um (S)-2-Piperidin-2-ylethanol als freie Base zu erhalten.

9. Das Trennverfahren nach einem der Ansprüche 1 bis 8, wobei das ausgefallene Salz in eine Reaktionsmischung eingebracht wird, die eine Base enthält, wodurch (S)-2-Piperidin-2-ylethanol als freie Base *in situ* in der Reaktionsmischung erhalten wird.

10. Das Trennverfahren nach einem der Ansprüche 1 bis 9, wobei das zur Verwendung in Schritt "a" gewählte Trennmittel N-Acetyl-L-leucin ist.

11. Das Trennverfahren nach einem der vorhergehenden Ansprüche, wobei das Trennmittel in der Lösung von Schritt "a" in einer Menge vorliegt, die ausreicht, um eine 5M Lösung des Trennmittels zu ergeben.

12. Das Trennverfahren nach einem vorhergehenden Anspruch, wobei die Mischung aus Lösungsmitteln, die in Schritt "a", dem Vereinschritt, verwendet wird, ein Volumenverhältnis von polarem aprotischem organischem Lösungsmittel (PAO-Lösungsmittel) : aliphatischem Alkohol von etwa 2:1 PAO-Lösungsmittel:aliphatischem Alkohol bis etwa 10:1 PAO-Lösungsmittel:aliphatischem Alkohol umfasst.

13. Das Trennverfahren nach Anspruch 12, wobei die verwendete Lösungsmittelmischung ein Volumenverhältnis von etwa 5:1 PAO-Lösungsmittel:aliphatischem Alkohol besitzt.

14. Das Trennverfahren nach einem vorhergehenden Anspruch, wobei das PAO-Lösungsmittel THF ist und eine Lösung umfasst, die eine ausreichende Menge an 2-Piperidin-2-ylethanol enthält, um eine Lösung zu ergeben, die wenigstens 2M in 2-Piperidin-2-ylethanol ist.

15. Das Trennverfahren nach einem vorhergehenden Anspruch, wobei die vereinten Methanol- und THF-Lösungen in Schritt "a" bei einer Temperatur von etwa 20°C bis etwa 60°C gehalten werden.

16. Das Trennverfahren nach einem vorhergehenden Anspruch, wobei die vereinten Methanol- und THF-Lösungen in Schritt "a" bei einer Temperatur von etwa 35°C bis etwa 40°C gehalten werden.

17. Das Trennverfahren nach einem vorhergehenden Anspruch, wobei die vereinte und verdünnte Lösung von Schritt "b" auf eine Temperatur von etwa 30°C bis zu einer Temperatur unterhalb der Rückflusstemperatur der Lösungsmittelmischung erwärmt wird.

18. Das Trennverfahren nach einem vorhergehenden Anspruch, wobei die vereinte und verdünnte Lösung von Schritt "b" auf eine Temperatur von etwa 50°C bis etwa 55°C erwärmt wird.

19. Das Trennverfahren nach einem vorhergehenden Anspruch, wobei Schritt "e", der Reinigungsschritt, durch Kristallisation des Niederschlags von Schritt "d" durchgeführt wird, indem eine Mischung aus 1 Vol.-Äquiv. Methanol und 20 Vol.-Äquiv. Acetonitril , in der das Salz gelöst ist, auf eine Temperatur von etwa 55°C erwärmt und auf eine Temperatur von etwa 15°C abgekühlt wird.

20. Das Trennverfahren nach einem vorhergehenden Anspruch, wobei Schritt "e", der Reinigungsschritt", durch Aufschlämmen des Niederschlages von Schritt "d" in einer Mischung aus 1 Vol.-Äquiv. Methanol und 20 Vol.-Äquiv. Acetonitril und Erwärmen der Aufschlämmung auf eine Temperatur von etwa 55°C, Beibehalten dieser Temperatur so lange, bis die Verunreinigungen gelöst sind, und Abkühlen auf eine Temperatur von etwa 15°C durchgeführt wird.

21. Ein Verfahren zur Herstellung von Pyrazol[1,5-a]pyrimidin-7-ylamino-Verbindungen der Formel III 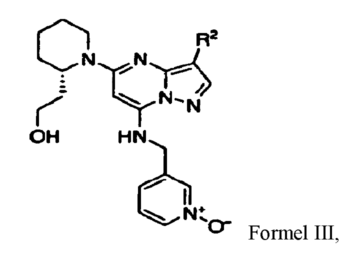 wobei R² eine lineare, verzweigte oder cyclische Alkylgruppe ist, wobei das Verfahren umfasst:
(a) Vereinen einer Lösung, die ein polares aprotisches organisches Lösungsmittel und eine Mischung aus Isomeren von 2-Piperidin-2-ylethanol enthält, mit einer Lösung aliphatischen Alkohols, die ein Aminosäure-Trennmittel, ausgewählt aus N-Acetyl-L-leucin und N-Acetyl-L-methionin, enthält,
(b) Vermischen der vereinten Lösungen mit zusätzlichen Mengen des polaren aprotischen organischen Lösungsmittels, und Erwärmen der Mischung auf eine Temperatur, die unterhalb des Siedepunktes eines jeden Lösungsmittels in der Mischung liegt und die ausreicht, um die Auflösung jeglicher Feststoffe, die in der Mischung vorliegen, zu fördern,
(c) Abkühlen der Mischung auf eine Temperatur, die es möglich macht, dass das Salz der Verbindung G1a aus der Mischung ausfällt, 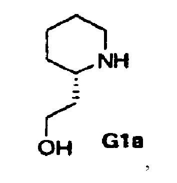
(d) Isolieren des in Schritt "c" gebildeten ausgefallenen Salzes,
(e) Reinigen des Salzes aus Schritt "c" durch Aufschlämmen des Salzes in einer, bezogen auf das Volumen, 10:1-Mischung aus Acetonitril (ACN) und Methanol,
(f) Freisetzen der Freie-Base-Form des Alkohols G1a aus dem in dem Reinigungsschritt "e" hergestellten Salz und
(g) Umsetzen des Alkohols in Form der freien Base aus Schritt "e" mit der Verbindung der Formel G1

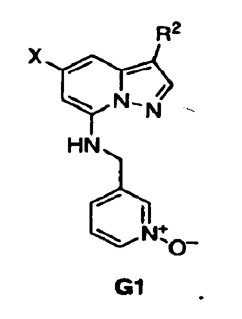

22. Das Verfahren nach Anspruch 21, wobei "R²" ein Ethylsubstituent ist und X Cl⁻ ist.

23. Das Verfahren nach einem der Ansprüche 21 bis 22, wobei das polare aprotische organische Lösungsmittel in Schritt "a" ausgewählt ist aus Tetrahydrofuran (THF), Acetonitril (ACN), Ethylacetat und Aceton.

24. Das Verfahren nach einem der Ansprüche 21 bis 23, wobei der aliphatische Alkohol in Schritt "a" ausgewählt ist aus aliphatischen Alkoholen mit 5 Kohlenstoffatomen oder weniger.

25. Das Verfahren nach einem der Ansprüche 21 bis 24, wobei der Freisetzungsschritt "f' durch Vereinen des isolierten getrennten Salzes mit einer Reaktionsmischung in Gegenwart einer Base und Freisetzen des Alkohols in Freie-Base-Form *in situ* durchgeführt wird.

26. Das Verfahren nach einem der Ansprüche 21 bis 24, wobei der Freisetzungsschritt "f' durch Behandeln des isolierten getrennten Salzes mit einer Base, Auskristallisieren des Alkohols als freie Base aus der Lösung und Isolieren der Kristalle der freien Base durch Filtration durchgeführt wird.

27. Ein Verfahren zum Abtrennen des S-Isomers von 2-Piperidin-2-ylethanol von einer Isomerenmischung, wobei das Verfahren umfasst:
(a) Vereinen einer Tetrahydrofuran(THF)-Lösung, die eine Mischung aus Isomeren von 2-Piperidin-2-ylethanol enthält, mit einer Methanollösung, die N-Acetyl-L-leucin enthält,
(b) Vermischen der vereinten Lösungen mit zusätzlichem THF und Erwärmen der Mischung auf eine Temperatur, die unterhalb des Siedepunktes eines jeden Lösungsmittels in der Mischung liegt,
(c) Abkühlen der Mischung auf eine Temperatur, die das Ausfällen von Salz aus der Mischung ermöglicht,
(d) gegebenenfalls Isolieren des in Schritt "c" gebildeten ausgefallenen Salzes und
(e) gegebenenfalls Umkristallisieren des Salzes.

28. Das Verfahren nach Anspruch 1, wobei das aprotische organische Lösungsmittel THF ist.

29. Das Verfahren nach den Ansprüchen 1 und 28, wobei der in Schritt "a" verwendete aliphatische Alkohol ein Alkohol mit 5 Kohlenstoffatomen oder weniger ist.

30. Das Verfahren nach Anspruch 29, wobei der aliphatische Alkohol Methanol ist.

31. Das Verfahren nach Anspruch 6, wobei der Umkristallisationsschritt "e", wenn er durchgeführt wird, wiederholt wird, bis die erwünschte Enantiomerenreinheit erreicht ist.

32. Das Verfahren nach Anspruch 31, ferner umfassend die Schritte:
(a) Bereitstellen einer Mischung, umfassend das Wiederauflösen oder Suspendieren des ausgefallenen Salzes in einem Lösungsmittel, und
(b) Behandeln der Mischung aus Schritt "a" mit einer Base, um (S)-2-Piperidin-2-ylethanol in Freie-Base-Form zu erhalten.

## Revendications

1. Procédé de résolution de l'isomère S de 2-pipéridin-2-yl-éthanol d'un mélange d'isomères, le procédé comprenant:
(a) combiner une solution comprenant un solvant organique aprotique polaire et un mélange d'isomères de 2-pipéridin-2-yl-éthanol, avec une solution d'alcool aliphatique contenant un agent de résolution à base d'acides aminés, sélectionné parmi la N-acétyl-L-leucine et la N-acétyl-L-méthionine;
(b) mélanger les solutions combinées avec des quantités supplémentaires du solvant organique aprotique polaire et chauffer le mélange à une température inférieure à la température d'ébullition d'un solvant ou de l'autre dans le mélange, suffisante pour favoriser la dissolution de tout solide présent dans le mélange;
(c) refroidir le mélange à une température qui permet la précipitation du sel de celui-ci;
(d) optionnellement isoler le sel précipité formé à l'étape "c"; et
(e) optionnellement purifier le sel isolé de l'étape "d" par recristallisation ou mise en suspension avec un solvant purifiant.

2. Procédé selon la revendication 1, dans lequel le solvant aprotique polaire utilisé à ladite étape "a" de "combinaison" est un solvant sélectionné parmi le tétrahydrofurane (THF), l'acétonitrile (ACN), l'acétone et l'acétate d'éthyle et des mélanges de deux ou plus de ceux-ci.

3. Procédé selon la revendication 2, dans lequel ledit solvant aprotique polaire est le THF.

4. Procédé selon l'une quelconque des revendications 1 à 3, dans lequel l'étape "e" de recristallisation, lorsque effectuée, est effectuée dans un solvant mélangé.

5. Procédé selon la revendication 4, dans lequel ledit solvant mélangé est un mélange de solvant OAP sélectionné parmi l'acétonitrile et le THF et d'un alcool aliphatique ayant 5 atomes de carbone ou moins.

6. Procédé selon la revendication 5, dans lequel le mélange comprend 19 volumes d'acétonitrile et 1 volume de méthanol.

7. Procédé selon la revendication 1, dans lequel ladite étape "e" de recristallisation, lorsque effectuée, est répétée jusqu'à l'obtention de la pureté énantiomérique désirée.

8. Procédé selon une partie quelconque de la revendication 1, comprenant en outre les étapes consistant à:
(a) fournir un mélange comprenant redissoudre ou mettre en suspension le sel précipité dans un solvant; et
(b) traiter le mélange de l'étape "a" avec une base pour donner la base libre de (S)2-pipéridin-2-yl-éthanol.

9. Procédé de résolution selon l'une quelconque des revendications 1 à 8, dans lequel le sel précipité est introduit dans un mélange de réaction contenant une base, donnant ainsi la base libre de (S)2-pipéridin-2-yl-éthanol *in situ* dans le mélange de réaction.

10. Procédé de résolution selon l'une quelconque des revendications 1 à 9, dans lequel l'agent de résolution sélectionné pour être utilisé à l'étape "a" est la N-acétyl-L-leucine.

11. Procédé de résolution selon l'une quelconque des revendications précédentes, dans lequel l'agent de résolution est présent dans la solution de l'étape "a" en une quantité suffisante pour fournir une solution 5 M d'agent de résolution.

12. Procédé de résolution selon l'une quelconque des revendications précédentes, dans lequel le mélange de solvants de l'étape "a", l'étape de combinaison, comprend un rapport volumétrique de solvant organique aprotique polaire (solvant OAP):alcool aliphatique d'environ 2:1 de solvant OAP:alcool aliphatique, à environ 10:1 de solvant OAP:alcool aliphatique.

13. Procédé de résolution selon la revendication 12, dans lequel le mélange de solvants utilisés comprend un rapport volumétrique de solvant OAP:alcool aliphatique d'environ 5:1.

14. Procédé de résolution selon l'une quelconque des revendications précédentes, dans lequel le solvant OAP est du THF et comprend une solution qui contient une quantité de 2-pipéridin-2-yl-éthanol suffisante pour donner une solution de 2-pipéridin-2-yl-éthanol de 2 M au moins.

15. Procédé de résolution selon l'une quelconque des revendications précédentes, dans lequel les solutions de méthanol et de THF combinées à l'étape "a", sont maintenues à une température d'environ 20°C à environ 60°C.

16. Procédé de résolution selon l'une quelconque des revendications précédentes, dans lequel les solutions de méthanol et de THF combinées à l'étape "a", sont maintenues à une température d'environ 35°C à environ 40°C.

17. Procédé de résolution selon l'une quelconque des revendications précédentes, dans lequel la solution combinée et diluée de l'étape "b" est chauffée à une température d'environ 30°C à une température inférieure à la température de reflux du mélange de solvants.

18. Procédé de résolution selon l'une quelconque des revendications précédentes, dans lequel la solution combinée et diluée de l'étape "b" est chauffée à une température d'environ 50°C à environ 55°C.

19. Procédé de résolution selon l'une quelconque des revendications précédentes, dans lequel l'étape "e" de purification est effectuée par cristallisation du précipité de l'étape "d" en chauffant un mélange de 1 volume équivalent de méthanol et de 20 volumes équivalents d'acétonitrile dans lequel le sel est dissous à une température d'environ 55°C et en le refroidissant à une température d'environ 15°C.

20. Procédé de résolution selon l'une quelconque des revendications précédentes, dans lequel l'étape "e" de purification est effectuée en mettant le précipité de l'étape "d" en suspension dans un mélange de 1 volume équivalent de méthanol et de 20 volumes équivalents d'acétonitrile et en chauffant la suspension à une température d'environ 55°C, en la maintenant à la température pendant un certain temps pour dissoudre les impuretés et en la refroidissant à une température d'environ 15°C.

21. Procédé de préparation de composés de pyrazolo[1,5-a]pyrimidin-7-yl-amino de la Formule III 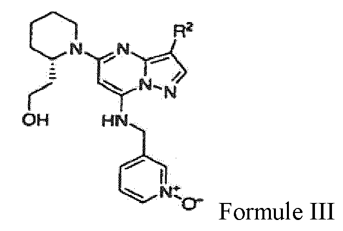 où R² est un groupe alkyle linéaire, ramifié ou cyclique, le procédé comprenant:
(a) combiner une solution comprenant un solvant organique aprotique polaire et un mélange d'isomères de 2-pipéridin-2-yl-éthanol avec une solution d'alcool aliphatique contenant un agent de résolution à base d'acides aminés, sélectionné parmi la N-acétyl-L-leucine et la N-acétyl-L-méthionine;
(b) mélanger les solutions combinées avec des quantités supplémentaires du solvant organique aprotique polaire et chauffer le mélange à une température inférieure à la température d'ébullition d'un solvant ou de l'autre dans le mélange, suffisante pour entraîner la dissolution de tout solide présent dans le mélange;
(c) refroidir le mélange à une température qui permet au sel d'un composé G1a de se précipiter de celui-ci, 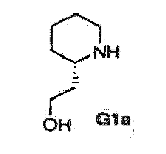
(d) isoler le sel précipité formé à l'étape "c";
(e) purifier le sel de l'étape "c" en mettant le sel en suspension dans un mélange d'acétonitrile (ACN) et de méthanol de 10:1;
(f) libérer l'alcool sous forme de base libre de G1a du sel préparé à l'étape "e" de purification; et
(g) mettre l'alcool sous forme de base libre de l'étape "e" à réagir avec le composé de la Formule G1,

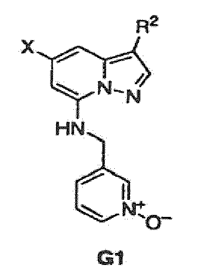

22. Procédé selon la revendication 21, dans lequel "R²" est un substituant éthyle et X est Cl⁻.

23. Procédé selon l'une quelconque des revendications 21 à 22, dans lequel le solvant organique aprotique polaire de l'étape "a" est sélectionné parmi le tétrahydrofurane (THF), l'acétonitrile (ACN), l'acétate d'éthyle et l'acétone.

24. Procédé selon l'une quelconque des revendications 21 à 23, dans lequel l'alcool aliphatique à l'étape "a" est sélectionné parmi des alcools aliphatiques ayant 5 atomes de carbone ou moins.

25. Procédé selon l'une quelconque des revendications 21 à 24, dans lequel ladite étape "f" de libération est effectuée en combinant le sel résous isolé avec un mélange de réaction en présence d'une base et en libérant l'alcool sous forme de base libre *in situ.*

26. Procédé selon l'une quelconque des revendications 21 à 24, dans lequel ladite étape "f" de libération est effectuée en traitant le sel résous isolé avec une base, en cristallisant l'alcool sous forme de base libre de la solution et en isolant les cristaux de base libre par filtration.

27. Procédé de résolution de l'isomère S de 2-pipéridin-2-yl-éthanol d'un mélange d'isomères, le procédé comprenant:
(a) combiner une solution de tétrahydrofurane (THF) contenant un mélange d'isomères de 2-pipéridin-2-yl-éthanol avec une solution de méthanol contenant de la N-acétyl-L-leucine;
(b) mélanger les solutions combinées avec du THF supplémentaire et chauffer le mélange à une température inférieure à la température d'ébullition d'un solvant ou de l'autre dans le mélange;
(c) refroidir le mélange à une température qui permet la précipitation du sel de celui-ci;
(d) optionnellement isoler le sel précipité formé à l'étape "c"; et
(e) optionnellement recristalliser le sel.

28. Procédé selon la revendication 1, dans lequel le solvant organique aprotique est du THF.

29. Procédé selon les revendications 1 et 28, dans lequel l'alcool aliphatique utilisé à l'étape "a" est un alcool ayant 5 atomes de carbone ou moins.

30. Procédé selon la revendication 29, dans lequel l'alcool aliphatique est du méthanol.

31. Procédé selon la revendication 6, dans lequel l'étape "e" de recristallisation, lorsque effectuée, est répétée jusqu'à l'obtention de la pureté énantiomérique désirée.

32. Procédé selon une partie quelconque de la revendication 31, comprenant les étapes consistant à:
(a) fournir un mélange comprenant redissoudre ou mettre en suspension le sel précipité dans un solvant; et
(b) traiter le mélange de l'étape "a" avec une base pour donner la base libre de (S)2-pipéridin-2-yl-éthanol.
